# EUROPEAN PATENT SPECIFICATION

(11) **EP 2 285 242 B1**
(45) Date of publication and mention of the grant of the patent: **01.05.2013**
(21) Application number: 09761742.7
(22) Date of filing: 10.06.2009
(51) Int. Cl.: A23L 1/30, A23L 1/00, A23D 7/06, C11B 5/00, A61K 36/53, A61K 31/201, A61K 31/202

(54) **PLANT EXTRACT AND PUFA COMBINATIONS**
PFLANZENEXTRAKT UND MEHRFACH UNGESÄTTIGTER FETTSÄUREN KOMBINATIONEN
COMBINAISONS D'EXTRAIT VEGETAL ET D'ACIDES GRAS POLYINSATURES

(30) Priority: 10.06.2008 EP 08010506; 12.06.2008 EP 08010653
(43) Date of publication of application: 23.02.2011
(73) Proprietor: DSM IP Assets B.V., 6411 TE Heerlen (NL)
(72) Inventor: GORALCZYK, Regina, 79639 Grenzach-Wyhlen (DE); ETHEVE, Stephane, F-68128 Village-Neuf (FR); PRUDENCE, Kevin, CH-4102 Binningen (CH); SCHWEIGERT, Loni, CH-4315 Zuzgen (CH)
(74) Representative: Schwander, Kuno
(86) International application number: PCT/EP2009/057189
(87) International publication number: WO 2009/150179

(56) References cited:
- WO-A-00/51576
- WO-A-2007/003762
- WO-A1-2008/017484
- WO-A1-2008/028584
- WO-A2-02/060510
- WO-A2-2008/012329
- FR-A1- 2 513 262
- US-A- 5 017 397
- US-A1- 2003 232 100
- US-A1- 2006 013 905
- US-B1- 6 200 601
- ZA-A- 9 904 696
- LAURENCE LIVERNAIS-SAETTEL: "Soybean oil" INTERNET ARTICLE, [Online] 2002, XP002543248 Retrieved from the Internet: URL:http://www.dietobio.com/dossiers/en/so y/oil.html> [retrieved on 2009-08-27]
- BAHLE ET AL.: "Oregano and rosemary extracts inhibit oxidation of long-chain n-3 fatty acids in Menhaden oil" JOURNAL OF FOOD SCIENCE, vol. 72, no. 9, 2007, pages C504-C508, XP002543249 cited in the application

## Description

### BRIEF DESCRIPTION OF THE INVENTION

The present invention relates to the combination of a plant extract, such as oregano extract and a polyunsaturated fatty acid or a derivative, such as an ethyl ester ("PUFA EE") which exhibits increased stability. In addition, it also relates to dietary, nutraceutical, and pharmaceutical compositions containing such combinations and/or their volatile components.

### BACKGROUND OF THE INVENTION

Many plant extracts are known to have various medicial properties or other health benefits. For example, oregano extract is known to have various mind/mood benefits, such as those described in WO08/017484 where oregano extract is shown to be useful in maintaining a balanced mood and mental performance and can act as an anti-depressant.

Over time, however, plant extracts have been observed to partially solidify, especially if these extracts are enriched in lipophilic contents. It is theorized that waxy substances present in the extract crystallize and precipitate out of solution. Active ingredients present in the plant extracts can be bound by these waxes thus become less bioavailable and/or degrade.

Long chain unsaturated fatty acids (PUFAs) are also known to have various health benefits. Numerous studies have shown that consumption of PUFAs can reduce the risk of degenerative diseases, cancer, cardiovascular disorders, depression, and various inflammatory diseases. One problem with PUFAs extracted from fish oil is that they are readily degraded in the presence of oxygen, producing an off-flavor and rancid smell. In order to stabilize them, various chemical preservatives can be used. However, health conscious consumers tend to prefer all-natural ingredients for their food.

Oregano extract has been used previously as an antioxidant preservative. Economou et al 1991 J. Am Oil Chem Soc 68(2):109-113 found that a methanol extract of oregano was superior to other herbal extracts for preserving lard. Kulisic et al 2004 Food Chemistry 85: 633-640, found oregano extract was a less effective antioxidant than ascorbic acid, but 5 comparable with α-tocopherol and the synthetic antioxidant butylated hydroxytoluene (BHT). See also Sahin et al 2004 Food Control 15: 549-557 where a hydrodistilled essential oil of oregano which had caryophyllene and spathulenol, germacrene-D and α-terpineol as its main constituents was tested, as was a methanol extract. Both had antimicrobial activities and were anti-oxidants. Botsoglou et al 2002 Meat Science 62: 259-10 265 found that when chickens were fed a diet that included 100 mg oregano essential oil/kg feed for 38 days, there was less lipid oxidation in raw and cooked meat, although better results were obtained with a dietary supplement of α-tocopheryl acetate.

FR-A- 2 513 262 (US 4, 525, 306) discloses capsules containing oils and fats to which an herbal antioxidant is added at an amount of 0.01 to 10%. Also, a phospholipid and an alcohol is present to improve the antioxidation effect.

The use of oregano extracts specifically to preserve PUFAs has also been investigated with mixed results. For example, Bhale et al 2007 *J*. *Food Sci.* 72(9):C504-508 compared the ability of a methanol extract of oregano to a rosemary extract to inhibit oxidation. Oregano extract was found to be superior to rosemary extract, but only at high temperatures and at higher concentration (around 5%). At lower temperatures, and at lower concentrations, the rosemary extract was superior. The PUFAs tested in this reference were from menhaden oil. Menhaden oil is known to contain only about 30% omega-3 long -chain unsaturated fatty acids such as eicosapentaenoic acid (EPA) and docosahexaenoic acid (DHA).

As the EPA content of PUFAs seems to be associated with a number of its healthful benefits, it would be desirable to produce a formulation of PUFAs and plant extracts where both the PUFAs and plant extracts were stable, and where both were present in sufficient amounts to be bioactive.

### DETAILED DESCRIPTION OF THE INVENTION

This invention relates to a stable combination of PUFAs and an oregano extract, which exhibits increased storage stability, wherein the concentrations of the PUFAs is at least 8% and the oregano extract concentration is at least 15%.

The combination is a mixture of oregano extract and PUFA ethyl esters, wherein both components are present in high concentrations, ie at least 8% (by weight) for the PUFA ethyl esters and at least 15% (by weight) for the oregano extract. This combination exhibits surprising stability and has a long shelf life.

This invention also relates to the use of the combination of oregano extracts and/or their volatile components and polyunsaturated fatty acid ethyl esters (hereinafter "PUFA EE/oregano extracts") for the manufacture of various nutraceuticals, dietary supplements and pharmaceutical compositions. In preferred embodiments, the PUFA EE/oregano extracts are present in a capsule.

The amount (by weight) of oregano extract in the composition is at least 15%, and may extend up further to at least 25%, or to at least 50% without exhibiting any pro-oxidative effects which are typically seen in high concentrations of conventional antioxidants.

### BRIEF DESCRIPTIONS OF THE FIGURES

FIGURE 1 shows the oxidation of ROPUFA '75' n-3 EE oil in varying concentations of oregano extract over time using an accelerated oxidation test, which is based on oxidation with oxygen under pressure. The consumption of oxygen is determined by pressure drop. The X-Axis is the number of hours; Y-Axis is pressure.
FIGURE 2 shows that the oxidation of ROPUFA '75' n-3 EE oil (0% Oregano extract) is faster when it is pure than when mixed with Oregano extracts at different concentrations.

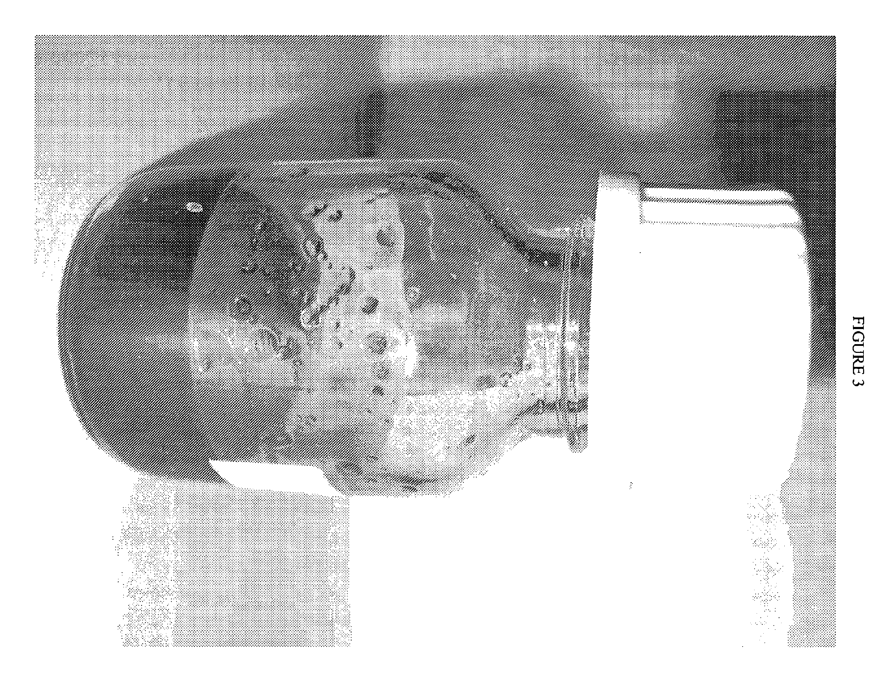

FIGURE 3 shows a vial of an oregano extract which exhibits waxing.

Unlike some conventional antioxidants such as ascorbic acid, which at higher concentrations can exhibit a pro-oxidation activity, the oregano extract retains its antioxidant capabilities at high concentrations. Further, the PUFA EEs are believed to stabilize the oregano oil over a long period, to prevent a waxy precipitation which has hitherto been observed.

### PUFAs

The PUFAs which can be used in this invention also includes PUFA derivatives, such as esters. Other derivatives include palmitic esters or other edible esters. One particularly preferred PUFA derivative is PUFA ethyl ester (PUFA EE). Also included in this invention are PUFA triglycerides (preferably a minimum 25% of PUFA, more preferably 30% PUFA, especially those which are rich in EPA.

The PUFAs which are preferably used in this invention may be from any source, with the proviso that when oregano extract is the plant extract, the PUFAs are not from menhaden oil. It may include marine oils, such as tuna, salmon, squid, krill (and specifically excluding menhaden oil *Brevoortia sp.* and *Ethmidium sp*.) but it can also include other sources of long chain n-3 fatty acids, such as those produced naturally or through genetically modified organisms such as plants, bacteria, fungi (especially yeast) or algae.

In preferred embodiments, the PUFAs of this invention contain a high amount, i.e. at least 30%, and preferable higher, of eicosapentaenoic acid or its ethyl ester, docosahexaenoic acid or its ethyl ester, and mixtures thereof. Such PUFA EEs are commercially available, such as the mixture sold by DSM Nutritional Products Ltd., Basel Switzerland under the Trademark ROPUFA 75. Commercially available PUFAs often contain a small amount of rosemary extract present as an antioxidant. In accordance with this invention, it has been found that there is no adverse effect in combining the plant extracts of this invention with the minor amount of rosemary extract present in the PUFAs.

### Plant extracts and their volatile components

The plant extract used in this invention is oregano. "Extract" as used herein is meant to encompass a broad category of plant originating material. For example, an extract may be any type of extract which is suitable for food use, such as aqueous extracts, steam extracts, plant oils, organic solvent extracts which are permitted in food (in particular ethanol extracts, propylene glycol extracts, ethyl acetate extracts, and supercritical fluid carbon dioxide (SF CO₂), etc. "Extract" also includes distillates, concentrates and other mixtures of plant active ingredients.

Oregano species are known to vary widely in their chemical makeup. See, for example, Vokou et al 1993 Biochemical Systematics and Ecology 21 (2):287-295 which describes the variation of essential oil in *O*. *vulgare* ssp. *hirtum* grown at twenty three localities. Due to this great variability, the botanic source of the extract is not as important to this invention as the chemical content of the extract. Thus, oregano extracts may be of any origin from any plant (whole plant or parts thereof) belonging to the genera *Origanum* as long as it contains carvacol and thymoquinone together with other volitiles as at a sufficient level to be effective, as detailed below.

Examples of plants from the genus *Origanum* which are preferred for use in this invention are *O*. *vulgare* and its subspecies and/or *O*. *minutiflorum.* Other species which may be used are: *O. majorana, O*. *dictamus, O*. *creticum, O. x majoricum, O*. *aureum, O*. *compactus, O*. *syriaca, O*. *tytthantum, O*. *heracleoticum, O*. *smyrnaeum, O*. *virens,* and *O. hirtum.*

Preferred are extracts containing a high proportion of at least one of their volatile components. More preferred are oregano extracts containing at least a total of 65 weight-% of volatile components as mentioned above, based on the total weight of the extract. Completely natural extracts may be fortified with at least one specific volatile component thereof.

Preferred oregano extracts in the context of the present invention are those wherein:
- the oregano extract comprises at least 30 weight-% of carvacrol,
- the oregano extract comprises at least 50 weight-% of carvacrol, and
- more preferably wherein the oregano extract comprises at least 55 weight-% of carvacrol, based on the weight of the oregano extract.

Also preferred are oregano extracts are oregano extracts which comprise thymoquinone in an amount in the range of from 0 to 30 weight-%,
- preferably wherein the oregano extract comprises at least 1 weight-% of thymoquinone,
- more preferably wherein the oregano extract comprises at least 2 weight-% of thymoquinone,
- more preferably wherein the oregano extract comprises at least 4 weight % of thymoquinone,
- most preferably wherein the oregano extract comprises thymoquinone in a range of from 4 to 30 weight-%, based on the weight of the oregano extract.

Other preferred oregano extracts are those wherein the oregano extract comprises:
- at least 50 weight-% of carvacrol and from 0 to 25 weight-%, of thymoquinone,
- preferably wherein the oregano extract comprises at least 50 weight-% of carvacrol and at least 1 weight-% of thymoquinone;
- more preferably wherein the oregano extract comprises at least 55 weight-% of carvacrol and at least 2 weight-% of thymoquinone, or
- wherein the oregano extract comprises at least 55 weight-% carvacrol and at least 4 weight -% thymoquinone, based on weight of the oregano extract.

Preferred oregano extracts are obtained by an extraction with the use of SF CO₂. Such extracts have the advantage that they should not contain any organic solvent residues, no proteins and no heavy metals. If desired, an extraction with supercritical carbon dioxide may be followed by a second supercritical fluid CO2-extraction step to reduce waxes and selectively enrich the volatiles.

The oregano extracts or their volatile components can be of natural or synthetic or mixed (viz. partly natural, partly synthetic) origin, i.e., the active components can be obtained by extraction of plants and fractionation, or they can be chemically synthesized and, if desired, mixed together with plant extracts in any desired quantities. They can be prepared and used in any desired purities and concentrations, e.g. as solutions containing them in concentrations as low as, e.g., 10% (w/w) or less, or up to nearly 100% (w/w).

### PUFAs

The PUFAs of this invention preferably contain a high concentration of n-3 polyunsaturated fatty acid derivatives; preferably they are in the form of esters; preferably in the form of eicosapentaenoic acid ethyl ester (EPA EE) and docosahexaenoic acid ethyl ester (DHA EE). In preferred embodiments, the PUFAs are present at a concentration of at least 60 weight % in the preparation, more preferably in a concentration of at least 70 weight %; and most preferably in an amount of at least 75 weight %. The PUFA preparation may contain further carrier oils as required.

PUFA EEs compositions are highly vulnerable to oxidation, and are often supplied treated with anti-oxidants such as tocopherols, citric acid, rosemary extract and the like. These components may also be present in the mixtures of this invention. The ability of oregano extract to stabilize PUFAs, and especially EPA is observed regardless of the presence or absence of these other anti-oxidants.

It has been surprisingly found that oregano extract can specifically stabilize EPA EE. This is significant, as many of the health benefits of PUFAs come from the presence of EPA (or its derivative, EPA EE). Thus, in preferred embodiments of this invention, EPA or a derivative such as EPA EE makes up a higher percentage of the PUFA mixture than other PUFAs such as DHA (or DHA EE).

### COMBINATIONS

In preferred embodiments of this invention, the oregano extract and PUFA EEs are present in the ratio (by weight %) of from 15:85 to 92:8. In more preferred embodiments, the ratio of oregano is at least 8% of the mixture, preferably at least about 25%, and may be at least about 50%. These ratios provide two aspects of this invention: there is stability without any pro-oxidative effect observed, and secondly, both the PUFA EEs and the oregano extract are present in high enough concentrations so that both exert bioactive effects on the person consuming the mixture.

Some anti-oxidants, such as Vitamin E, can become pro-oxidants when they are present at a high amount. See for example, Setiadi et al 2003 Theochem 620(2-3): 93-106.

The compositions of the present invention are preferably in the form of capsules. The capsules may be a soft gel capsule or a sealed hard shell capsule. The capsule can contain the PUFA EE/oregano extract combination. The capsules themselves may be made by any conventional method and will usually contain e.g. a matrix of (fish, swine, poultry, cow) gelatin, a vegetable matrix based on cellulosic raw materials (e.g. hydroxypropyl methylcellulose), or a starch or starch derived material.

In addition to the PUFA EE/oregano extract, the capsules according to the present invention may further contain conventional pharmaceutical/nutraceutical additives and adjuvants, excipients or diluents, including, but not limited to: gelatin of any origin, vegetable gums, ligninsulfonate, talc, sugars, starch, gum arabic, vegetable oils, polyalkylene glycols, flavoring agents, preservatives, stabilizers, emulsifying agents, buffers, lubricants, colorants, wetting agents, fillers, and the like. Generally the capsule will contain an oil to adjust the filling volume to the capsule size, and may include an excipient for increasing viscosity, as PUFA EE tends to have a rather low viscosity. If a viscosity increasing agent is present, it may be selected from any of the known viscosity increasing agents, such as bee wax, mineral waxes, plant-based waxes, colloidal silica or lecithin.

Typically, a beneficial daily dose for a human of oregano extract is from 10 mg to 300 mg. This may be taken in a single dose, or due to pharmacokinetics of the oregano extract, it may be advisable to ingest partial dosages several times per day. For someone taking capsules twice a day, the total amount of oregano delivered at each dosing time should thus range from 5 mg to 200 mg. If one were to consume a total of six capsules per day (3 capsules twice a day), then each capsule should contain approximately 1.5 to 50 mg oregano. Based on this, one of ordinary skill in the art can likewise determine amount appropriate for consuming 4, 2, or a single capsule per day. Preferably dose will be 30, 60, 120, or 180 mg, or ∼3.5%, 7%, 13% or 19% by weight of a dosage.

For PUFAs, the amount per capsule should range from 50-1000 mg, striving for a daily intake of at least 1000 to 6000 mg per day total triglycerides (including non-capsule dietary sources). For high n-3 PUFA EE formulations, the equivalent amount (i.e. has a higher concentration of triglycerides than naturally occurring), the amounts may be adjusted downwards accordingly, and the size/amount of capsules can be reduced. An additional advantage to the concentrated formulations is that they are more easily digested, and have less of a fishy aftertaste.

One preferred capsule of this invention will contain the mixture of 60 mg oregano extract and 750 mg PUFA EEs. This mixture will make up between 50-60% by weight of the total capsule contents. The remaining 50-40% will include other excipients and fillers, including one or more viscosity adjusters such as glycerol, silicon dioxide lecithin or wax(es).

### Examples

### EXAMPLE 1

### Preparation of Materials

### Oregano Extract

The dry leaves and flowers of *Origanum vulgare* or *O*. *minutiflorum* were extracted by supercritical fluid extraction with natural carbon dioxide. The extract was obtained from Flavex Naturextrakte GmbH.

The extract is a dark reddish-brown liquid with characteristic smell. The extract used (RV0141-73), was analyzed by Flavex with GC/MS system with 100% peak area method, and contains 85.3% essential oil.

The main components were: 79.9% peak area phenolic carvacrol and 6.44% peak area thymoquinone. Other compounds present are 2.4% peak area cymene, 2.8% peak area linalool, 2.3% peak area borneol, 1.9% peak area caryophyllene and a small amount of limonene, 4-terpineol and thymol.

ROPUFA '75' n-3 EE oil (UT07050003) is a yellowish oil liquid and is produced by DSM Nutritional Products GmbH. It contains at least 75% of n-3 polyunsaturated fatty acids (PUFAs) in the form of ethyl esters, predominantly as eicosapentaenoic acid (EPA EE) and docosahexaenoic acid (DHA EE). It is treated and stabilized with ascorbyl palmitate, tocopherols, citric acid and contains rosemary extract (about 1000 ppm).

### EXAMPLE 2

### OXIDATIVE STABILITY

The relative stability of lipid solutions samples towards oxidation was measured in an accelerated oxidation test, using the Oxipres apparatus. The initial conditions were set up to 70°C and 5-6 bars. The ML OXIPRES™ (Mikrolab Aarhus A/S, Denmark) apparatus is a modification of the bomb method, which is based on oxidation with oxygen under pressure. See, Gearhart 1957 J. Amer. Oil Chem. Soc 34: 427 and Stuckey et al J. Amer. Oil Chem. Soc. 35: 581 and Blankenship et al 1973 J. Amer. Oil Chem. Soc. 50:377-318. The test is accelerated when carried out at elevated pressure. The consumption of oxygen is determined by pressure drop in the bomb during the experiment.

The first experiment was conducted with five solution samples: Blank, ROPUFA '75' n-3 EE oil mixed with Oregano extract at different concentrations (0%, 8%, 25% and 100%). No specific quantitative analysis was performed during this experiment.

The second experiment was conducted with six solution samples: ROPUFA '75' n-3 EE oil mixed with Oregano extract at different concentrations (0%, 4%, 8%, 25%, 50% and 100%). For this experiment further quantitative and qualitative analysis were performed at T0 and Tfinal for each sample: quantitation of thymoquinone, carvacrol, ascorbyl palmitate, tocopherols (α-, γ- and δ-) and PUFA (as methyl ester).

### Results

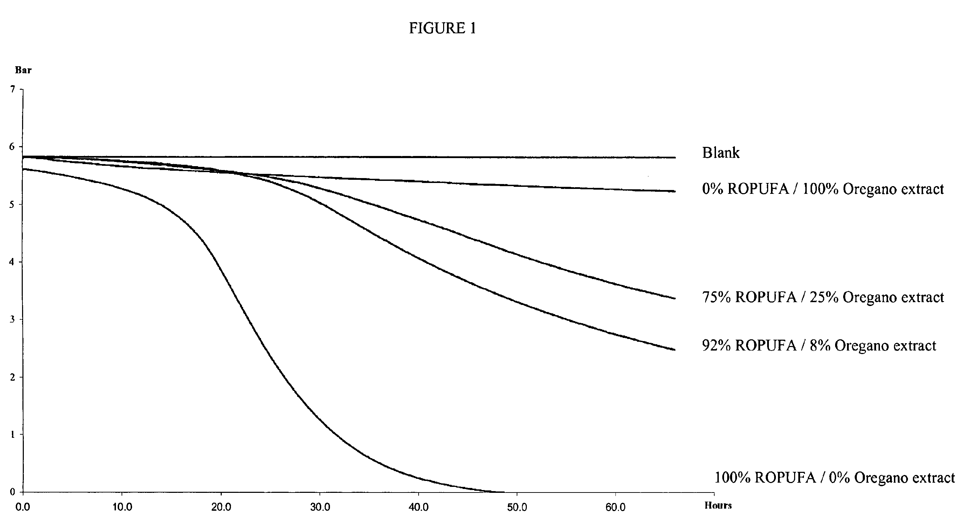

FIGURE 1 shows that the oxidation of pure ROPUFA '75' n-3 EE oil (0% oregano extract) is faster than with the mixed solution samples at different oregano extract concentrations (8% and 25%). After about 65 hours, the oxidation inhibition capability of oregano extract increases significantly with increasing concentration of oregano. The curve for the pure oregano extract (100%) does not significantly decrease compared to the blank sample, thus demonstrating that the pure oregano extract is stable.

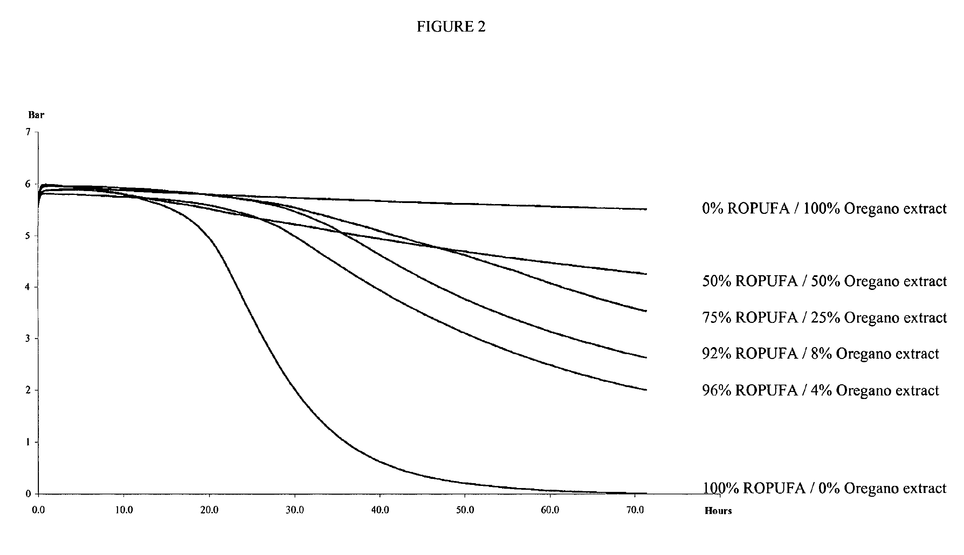

In agreement with the first experiment, FIGURE 2 shows that the oxidation of pure ROPUFA '75' n-3 EE oil (0% Oregano extract) is faster than with the mixed solution samples at different Oregano extract concentrations (4%, 8%, 25% and 50%). After about 70 hours, the oxidation inhibition capability of oregano extract increased significantly with increasing concentration of oregano extract. The curve of pure Oregano extract (100%) decreases not significantly, indicating the pure oregano extract is stable.

### EXAMPLE 3

### PUFA analysis

The identifications of EPA, DHA and DPA were done by comparing the retention times with authentic reference standards. Quantification was performed by applying an internal standard calibration with tricosanoic acid methyl ester.

In the Table 1, below, the results of the solution samples before and after the Oxipres experiment are shown. The determined concentrations (Cᵢₙₜᵢₐₗ) of EPA EE, DHA EE, DPA EE and total PUFA for each solution samples analyzed before the oxipres experiment is used as reference value to evaluate the stability of PUFA compounds after Oxipres experiment. From the results after the Oxipres experiment (C_{final}), the percentage of retained PUFAs (expressed as %accuracy = C_{final} / Cᵢₙᵢₜᵢₐₗ * 100) vs. the reference value is calculated for each solutions samples to observe the stability of PUFAs.

**Table 1 Analysis of PUFA - ROPUFA '75' n-3 EE oil mixed with Oregano extract at different concentrations. %Accuracy of an analytical procedure expresses the closeness of agreement between the value found (C_{final}) and an accepted reference value (Cᵢₙᵢₜᵢₐₗ).**

| | **EPA %accuracy** | **DHA %accuracy** | **DPA %accuracy** | **Total PFUA %accuracy** |
|---|---|---|---|---|
| **Sample 1** *100% Oregano extract* | <LOQ | <LOQ | <LOQ | **<*LOQ*** |
| **Sample 2** *50% Oregano extract* | *55.2* | *49.0* | *54.5* | **52.9** |
| **Sample 3** *25% Oregano extract* | *53.4* | *47.8* | *53.1* | **51.4** |
| **Sample 4** *8% Oregano extract* Not part of the invention | *53.5* | *47.1* | *51.6* | **51.1** |
| **Sample 5** *4% Oregano extract* | *46.7* | *43.6* | *49.4* | **45.8** |
| **Sample 6** *0% Oregano extract* | *41.6* | *41.4* | *65.6* | **43.5** |

In the above table, "EPA" refers to EPA EE; "DHA" refers to DHA EE, and "DPA" refers to DPA EE.

The oregano extract demonstrated significant capabilities in retarding total PUFA oxidation. In the pure ROPUFA '75' n-3 EE oil solution sample the total PUFA decreased to 43.5% of the initial concentration. The oxidation inhibition capability of oregano extract increased the stability of total PUFA significantly with increasing Oregano extract concentrations (4%, 8%, 25% and 50%) in the different solution samples respectively 45.8%, 51.1%, 51.4% and 52.9% of the initial value.

The retained EPA EE, DHA EE and DPA EE in the pure ROPUFA '75' n-3 EE oil decreased to 41,6%, 41.4% and 65.6% respectively of the initial concentration. EPA EE (329 mg/g) is the main component of ROPUFA '75' n-3 EE oil. EPA EE has higher increased stability with increasing oregano extract concentrations (4%, 8%, 25% and 50%) in the different solution samples respectively 46.7%, 53.5%, 53.4% and 55.2% of the initial value.

DHA EE (210 mg/g) is the second main component of ROPUFA '75' n-3 EE oil. DHA EE has a high increase of stability with increasing Oregano extract concentrations (4%, 8%, 25% and 50%) in the different solution samples respectively 43.6%, 47.1 %, 47.8% and 49.0 % of the initial value.

DPA EE (47.7 mg/g) is one of the lesser components of ROPUFA '75' n-3 EE oil. DPA EE also shows an increased oxidation inhibition capability of Oregano extract with increasing Oregano extract concentrations (4%, 8%, 25% and 50%) in the different solution samples respectively 43.6%, 47.1%, 47.8% and 49.0 % of the initial value.

### EXAMPLE 4

### Soft gel cap

Oblong soft gelatin capsules, size 16 (volume = 0.985), are prepared according to commonly known technologies and containing the following composition:

| **Ingredient** | **Amount per Capsule** |
|---|---|
| Oregano extract | 180 mg |
| PUFA EE | 750 mg |
| SiO2, colloidal | 30 mg |
| Palm oil | 50 mg |

Similar capsules to the above can also be made using 30, 60, or 120 mg oregano extract. Preferably these capsules are taken once or twice per day.

## Claims

1. A stable composition comprising an oregano extract and a polyunsaturated fatty acid (PUFA) or derivative of a PUFA, wherein the oregano extract is present in an amount of at least 15 % by weight and the PUFA or derivative of the PUFA comprises at least 30% eicosapentaenoic acid or its ethyl ester, docosahexaenoic acid or its ethyl ester, and mixtures thereof.

2. The composition according to Claim 1 where the PUFA is a PUFA ethyl ester.

3. The composition of Claim 1 or 2 wherein the oregano extract is obtained from plants of the genus *Origanum* by extraction with liquid carbon dioxide under supercritical conditions.

4. The composition of any of Claims 1-3 wherein the PUFA EE/oregano extract is obtained from *Origanum vulgare* and/or *Origanum minutiflorum.*

5. The composition of any one of claims 1-4 wherein the oregano extract contains a high portion of at least one of the volatile components.

6. The composition of claim 5 wherein the volatile components comprise carvacrol, thymol, thymoquinone and thymoquinol.

7. A composition according to any of Claims 1-6 which is a capsule.

8. A capsule according to Claim 7 containing at least 3.5% by weight oregano extract.

9. A food comprising the composition of any of Claims 1-6.

## Patentansprüche

1. Stabile Zusammensetzung, die einen Origanoextrakt und eine mehrfach ungesättigte Fettsäure (PUFA) oder ein PUFA-Derivat umfasst, wobei der Origanoextrakt in einer Menge von mindestens 15 Gew.-% vorliegt und die PUFA oder das PUFA-Derivat mindestens 30% Eicosapentaensäure oder Eicosapentaensäureethylester, Docosahexaensäure oder Docosahexaensäureethylester und Mischungen davon umfasst.

2. Zusammensetzung nach Anspruch 1, wobei es sich bei der PUFA um einen PUFA-Ethylester handelt.

3. Zusammensetzung nach Anspruch 1 oder 2, wobei der Origanoextrakt von Pflanzen der Gattung *Origanum* durch Extraktion mit flüssigem Kohlendioxid unter überkritischen Bedingungen erhalten wird.

4. Zusammensetzung nach einem der Ansprüche 1-3, wobei der PUFA-Ethylester/Origanoextrakt aus *Origanum vulgare* und/oder *Origanum minutiflorum* erhalten wird.

5. Zusammensetzung nach einem der Ansprüche 1-4, wobei der Origanoextrakt einen hohen Anteil an mindestens einer der flüchtigen Komponenten enthält.

6. Zusammensetzung nach Anspruch 5, wobei die flüchtigen Komponenten Carvacrol, Thymol, Thymochinon und Thymochinol umfassen.

7. Zusammensetzung nach einem der Ansprüche 1-6, bei der es sich um eine Kapsel handelt.

8. Kapsel nach Anspruch 7, die mindestens 3,5 Gew.-% Origanoextrakt enthält.

9. Nahrungsmittel, das die Zusammensetzung nach einem der Ansprüche 1-6 umfasst.

## Revendications

1. Composition stable comprenant un extrait d'origan et un acide gras polyinsaturé (PUFA) ou un dérivé d'un PUFA, **caractérisée en ce que** l'extrait d'origan est présent dans une quantité d'au moins 15 % en poids et le PUFA ou le dérivé du PUFA comprend au moins 30 % d'acide eicosapentaénoïque ou son ester éthylique, d'acide docosahexaénoïque ou son ester éthylique, et de mélanges de ceux-ci.

2. Composition selon la revendication 1, **caractérisée en ce que** le PUFA est un ester éthylique de PUFA.

3. Composition selon la revendication 1 ou 2, **caractérisée en ce que** l'extrait d'origan est obtenu à partir de plantes du genre *Origanum* par extraction avec du dioxyde de carbone liquide dans des conditions supercritiques.

4. Composition selon l'une quelconque des revendications 1 à 3, **caractérisée en ce que** l'EE de PUFA/extrait d'origan est obtenu à partir d'*Origanum vulgare* et/ou *Origanum minutiflorum.*

5. Composition selon l'une quelconque des revendications 1 à 4, **caractérisée en ce que** l'extrait d'origan contient une grande partie d'au moins l'un des composants volatils.

6. Composition selon la revendication 5, **caractérisée en ce que** les composants volatils comprennent le carvacrol, le thymol, la thymoquinone et le thymoquinol.

7. Composition selon l'une quelconque des revendications 1 à 6, **caractérisée en ce qu'**il s'agit d'une capsule.

8. Capsule selon la revendication 7, contenant au moins 3,5 % en poids d'extrait d'origan.

9. Aliment comprenant la composition selon l'une quelconque des revendications 1 à 6.
